# EUROPÄISCHE PATENTSCHRIFT

(11) **EP 0 623 102 B1**
(45) Veröffentlichungstag und Bekanntmachung des Hinweises auf die Patenterteilung: **24.06.1998**
(21) Anmeldenummer: 94900795.9
(22) Anmeldetag: 08.11.1993
(51) Int. Cl.: C07C 25/18, C09K 19/30, G02F 1/13

(54) **CYCLOBUTAN-BENZOL-DERIVATE**
CYCLOBUTANE-BENZOLE DERIVATIVES
DERIVES DE CYCLOBUTANE-BENZOL

(30) Priorität: 21.11.1992 DE 4239169
(43) Veröffentlichungstag der Anmeldung: 09.11.1994
(73) Patentinhaber: MERCK PATENT GmbH, 64293 Darmstadt (DE)
(72) Erfinder: POETSCH, Eike, D-64367 Mühltal (DE); BINDER, Werner, D-64807 Dieburg (DE); MEYER, Volker, D-64846 Gross-Zimmern (DE); FINKENZELLER, Ulrich, D-68723 Plankstadt (DE); RIEGER, Bernhard, D-64839 Münster (DE)
(86) Internationale Anmeldenummer: EP9303112
(87) Internationale Veröffentlichungsnummer: WO9412455

(56) Entgegenhaltungen:
- EP-A- 0 449 015
- WO-A-88/09322
- WO-A-91/08184
- DE-A- 4 027 923
- GB-A- 2 155 946
- JP-A- 2 085 243

## Beschreibung

Die Erfindung betrifft Cyclobutan-Benzol-Derivate der Formel I, 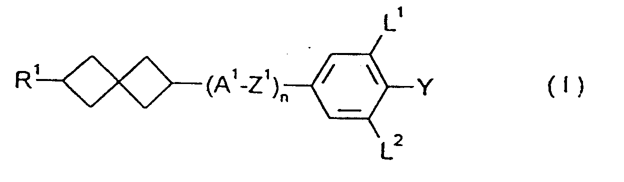 wobei
- R¹: Alkyl oder Alkenyl mit 1 bis 16 C-Atomen, worin auch eine oder mehrere CH₂-Gruppen durch -O-ersetzt sein können oder eine Gruppe der Formel 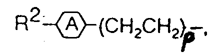 worin
- R²: Alkyl oder Alkenyl mit 1 bis 16 C-Atomen, 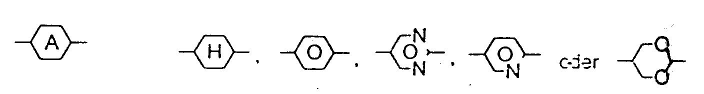 und
- p: 0 oder 1 bedeuten,
- A¹: jeweils unabhängig voneinander unsubstituiertes oder durch 1 bis2 Fluoratome substituiertes 1,4-Phenylen, worin auch eine oder zwei CH-Gruppen durch N ersetzt sein können, oder unsubstituiertes oder durch eine Cyanogruppe substituiertes 1,4-Cyclohexylen worin auch eine oder zwei CH₂-Gruppen durch O oder S ersetzt sein können, Thiadiazol-2,5-diyl, 1,4-Bi-cyclo[2,2,2]-octylen
bedeuten,
- L¹ und L²: jeweils unabhängig voneinander H oder F,
- Z¹: -CO-O-, -O-CO-, -CH₂O-, -OCH₂-, -CH₂CH₂-, -C≡C- oder eine Einfachbindung,
- Y: NCS, Halogen oder eine durch mindestens ein Fluor- und/oder Chloratom substituierte Alkyl-, Alkoxy-, Alkenyl oder Alkenyloxygruppe mit 1 bis 8 C-Atomen,
CN, oder Alkyl, oder Alkenyl mit bis zu 16 C-Atomen, worin auch 1 oder mehrere CH₂-Gruppen durch -O- ersetzt sein können,
- n: 0, 1 oder 2
bedeuten.

Die Erfindung betrifft weiterhin die Verwendung dieser Verbindungen als Komponenten flüssigkristalliner Medien sowie Flüssigkristall- und elektrooptische Anzeigeelemente, die die erfindungsgemäßen flüssigkristallinen Medien enthalten

Die Verbindungen der Formel I können als Komponenten flüssigkristalliner Medien verwendet werden, insbesondere für Displays, die auf dem Prinzip der verdrillten Zalle einschließlich deren hochverdrillten Varianten, wie z.B. STN oder SBE, dem Guest-Host-Effekt, dem Effekt der Deformation aufgerichteter Phasen oder dem Effekt der dynamischen Streuung beruhen

Der Erfindung lag die Aufgabe zugrunde neue stabile flüssigkristalline oder mesogene Verbindungen aufzufinden, die als Komponenten flüssigkristalliner Medien geeignet sind und insbesondere eine vergleichsweise geringe Viskosität besitzen sowie eine mittlere positive dielektrische Anisotropie

Es wurde nun gefunden, daß Verbindungen der Formel I als Komponenten flüssigkristalliner Phasen vorzüglich geeignet sind. Insbesondere verfügen sie über vergleichsweise niedere Viskositäten. Mit ihrer Hilfe lassen sich stabile flüssigkristalline Phasen mit breitem Mesophasenbereich vorteilhaften Werten für die optische und dielektrische Anisotropie erhalten, welche sich gleichzeitig durch sehr günstige Werte für den spezifischen Widerstand und niedrigen Viskositäten auszeichnen. Hierdurch lassen sich insbesondere bei Medien für Aktive-Matrix-Displays oder Supertwistdisplays deutliche Vorteile erzielen.

Aus der japanischen Offenlegungsschrift JP 02 085 243 sind Benzonitrilderivate der Formel 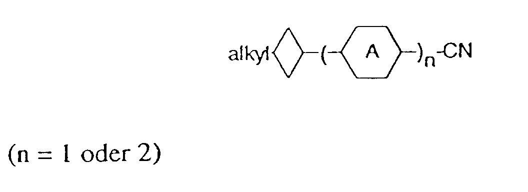 (n = 1 oder 2)
bekannt.

In EP-A 44 90 15 und DE-A 40 27 923 wird die Cyclobutangruppe als isolierter Baustein in der Seitenkette von flüssigkristallinen Verbindungen genannt, nicht aber als Bestandteil des mesogenen Moleküllkerns wie in den erfindungsgemäßen Verbindungen.

Aus der WO-A 88/09 322 sind Spiro[3.3]heptane als Komponenten flüssigkristalliner Phasen bekannt, jedoch findet sich kein Hinweis auf direkt mit dem mesogenen Kern verknüpfte Spiro[3.3]heptane oder auf deren Herstellung.

Von der allgemeinen Formel der GB-A 21 55 946 werden zwar direkt mit aromatischen Ringen verknüpfte Spiro[3.3]heptane umfaßt, ihre Herstellung wird jedoch nur in allgemeiner Form angegeben.

Weiterhin werden ähnliche Cyclobutan-Derivate beschrieben von:

DE-OS 37 17 484:

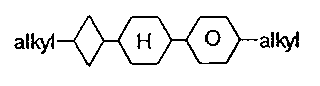

DE-OS 39 29 524:

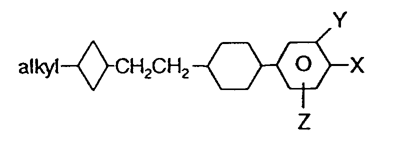

Von der allgemeinen Formel der WO 91/08184 werden Benzol-Derivate, welche eine 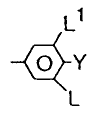 -Gruppe aufweisen, umfaßt.

Es sind dort jedoch keine Cyclobutan-Derivate beschrieben.

Weiterhin weisen die im Stand der Technik beschriebenen Verbindungen lediglich mittlere Werte der optischen Anisotropie auf, wohingegen die erfindungsgemäßen Verbindungen deutlich niedrigere optische Anistropien und Viskositäten besitzen.

### Gegenstand der Erfindung

Mit der Bereitstellung von Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Gegenstand der Erfindung sind somit die Verbindungen der Formel I.

Bevorzugte Ausführungsformen der vorliegenden Erfindung sind:
a) Derivate, worin L¹ und L² gleich sind und F bedeuten;
b) Derivate der Formel I3, 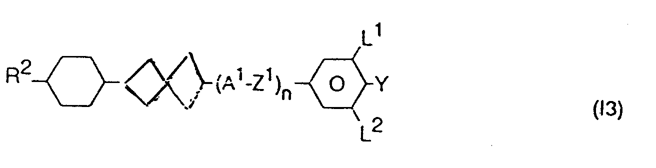 insbesondere der Formel I3a 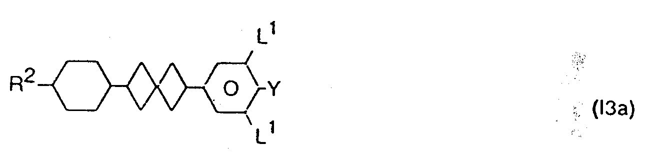 worin R², A¹, Z¹, L¹, L², n und Y die angegebene Bedeutung besitzen.

Gegenstand der Erfindung ist weiterhin die Verwendung dieser Verbindungen als Komponenten flüssigkristalliner Medien. Gegenstand der Erfindung sind ferner flüssigkristalline Medien mit einem Gehalt an mindestens einer Verbindung; sowie Flüssigkristallanzeigeelemente, insbesondere elektrooptische Anzeigeelemente, die derartige Medien enthalten, insbesondere Matrix-Flüssigkristallanzeigen.

### Bevorzugte Ausführungsformen

Der Einfachheit halber bedeuten im folgenden Cbu einen Rest der Formel 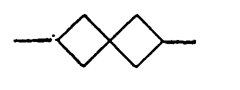 A³ einen Rest der Formel 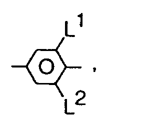 Cyc einen 1,4-Cyclohexylenrest, Dio einen 1,3-Dioxan-2,5-diylrest, Dit einen 1,3-Dithian-2,5-diylrest, Phe einen 1,4-Phenylenrest, PheF einen ein- oder zweifach durch Fluor substituierten 1,4-Phenylenrest, Pyd einen Pyridin-2,5-diylrest, Pyr einen Pyrimidin2,5-diylrest und Bi einen Bicyclo(2,2,2)-octylenrest, wobei Cyc unsubstituiert oder einfach durch CN substituiert sein kann. Phe kann unsubstituiert oder ein- oder zweifach durch F substituiert sein.

Die Verbindungen der Formel I umfassen dementsprechend Verbindungen mit zwei Ringen der Teilformel Ia:

R¹-Cbu-A³-Y Ia

Verbindungen mit drei Ringen der Teilformeln Ib bis Ig:

R¹-Cbu-A¹-A³-Y Ib

R¹-Cbu-A¹-Z¹-A³-Y Ic

R²-Cyc-Cbu-A³-Y Id

R²-Dio-Cbu-A³-Y Ie

R²-Cyc-CH₂CH₂-Cbu-A³-Y If

R²-Dio-CH₂CH₂-Cbu-A³-Y Ig

sowie Verbindungen mit vier Ringen der Teilformeln Ih bis Iq:

R¹-Cbu-A¹-A¹-A³-Y Ih

R¹-Cbu-A¹-Z¹-A¹-A³-Y Ii

R¹-Cbu-A¹-A¹-Z¹-A³-Y Ij

R¹-Cbu-A¹-Z¹-A¹-Z¹-A³-Y Ik

R²-Cyc-Cbu-A¹-A³-Y Il

R²-Dio-Cbu-A¹-A³-Y Im

R²-Cyc-Cbu-A¹-Z¹-A³-Y In

R²-Dio-Cbu-A¹-Z¹-A³-Y Io

R²-Cyc-CH₂CH₂-Cbu-A¹-A³-Y Ip

R²-Dio-CH₂CH₂-Cbu-A¹-A³-Y Iq

Darunter sind besonders diejenigen der Teilformeln Ia, Ib, Ic, Id, Ie, If, Ig, Ii und Il bevorzugt.

Die bevorzugten Verbindungen der Teilformel Ia umfassen diejenigen der Teilformeln Iaa und Iab:

R¹-Cbu-Phe-Y Iaa

R¹-Cbu-Phe-F-Y Iab

Darunter sind diejenigen der Formel Iab besonders bevorzugt.

Die bevorzugten Verbindungen der Teilformel Ib umfassen diejenigen der Teilformeln Iba bis Ibf:

R¹-Cbu-Phe-Phe-Y Iba

R¹-Cbu-Phe-PheF-Y Ibb

R¹-Cbu-Cyc-Phe-Y Ibc

R¹-Cbu-Cyc-PheF-Y Ibd

R¹-Cbu-PheF-Phe-Y Ibe

R¹-Cbu-PheF-PheF-Y Ibf Ibf

Die bevorzugten Verbindungen der Teilformel Ic umfassen diejenigen der Teilformeln Ica bis Ich:

R¹-Cbu-Phe-Z¹-Phe-Y Ica

R¹-Cbu-Phe-Z¹-PheF-Y Icb

R¹-Cbu-Cyc-Z¹-Phe-Y Icc

R¹-Cbu-Cyc-Z¹-PheF-Y Icd

R¹-Cbu-Dio-Z¹-Phe-Y Ice

R¹-Cbu-Dio-Z¹-PheF-Y Icf

R¹-Cbu-Pyd-Z¹-A³-Y Icg

R¹-Cbu-Pyr-Z¹-A³-Y Ich

Darunter sind diejenigen der Formeln Ica, Icb und Icc besonders bevorzugt.

Die bevorzugten Verbindungen der Teilformel Id umfassen diejenigen der Teilformeln Ida und Idb:

R²-Cyc-Cbu-Phe-Y Ida

R²-Cyc-Cbu-PheF-Y IDB

Die bevorzugten Verbindungen der Teilformel Ie umfassen diejenigen der Teilformeln Iea und Ieb:

R²-Dio-Cbu-Phe-Y Iea

R²-Dio-Cbu-PheF-Y Ieb Ieb

In den Verbindungen der vor- und nachstehenden Formeln sind die Reste L¹ und L² gleich oder verschieden voneinander; vorzugsweise sind sie gleich und bedeuten F.

Die Reste Cbu² und Cbu⁵ weisen die folgende jeweilige Bedeutung auf 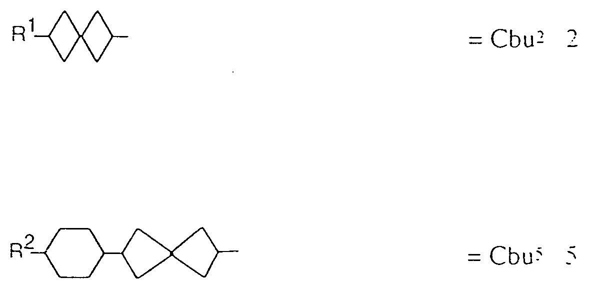 A¹ bedeutet bevorzugt Phe, Cyc, Pyr oder Dio. Bevorzugt enthalten die Verbindungen der Formel I nicht mehr als einen der Reste Bi, Pyd, Pyr, Dio oder Dit.

Bevorzugt sind auch Verbindungen der Formel I sowie aller Teilformeln, in denen A¹ ein- oder zweifach durch F substituiertes 1,4-Phenylen bedeutet. Insbesondere sind dies 2-Fluor-1,4-phenylen, 3-Fluor-1,4-phenylen und 2,3-Difluor-1,4-phenylen, 2,6-Difluor-1,4-phenylen sowie 3,5-Difluor-1,4-phenylen.

Die Gruppe A³-Y bedeutet vorzugsweise: 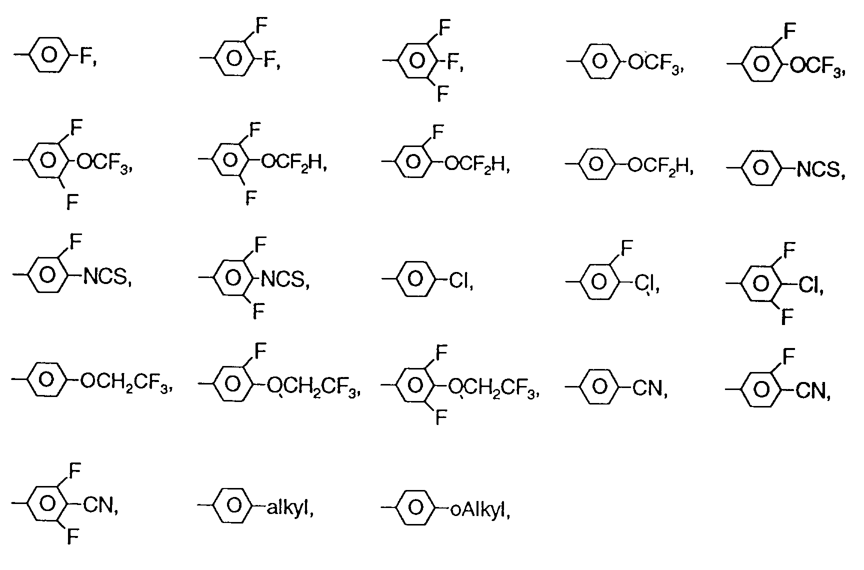 wobei Alkyl Alkyl mit 1 bis 16 C-Atomen bedeutet
und OAlkyl für Alkoxy mit 1 bis 15 C-Atomen steht.

Bevorzugt sind diejenigen Reste A³, worin mindestens eine der Liganden L¹ und L² F bedeutet, insbesondere worin bei L¹ und L² F bedeuten.

Z¹ bedeutet bevorzugt eine Einfachbindung, -CO-O-, -O-CO- und - CH₂CH₂-, in zweiter Linie bevorzugt -CH₂O-und -OCH₂-.

Falls R¹ oder Y einen Alkylrest bedeutet, so kann dieser geradkettig oder verzweigt sein. Vorzugsweise ist er geradkettig, hat 2, 3, 4, 5 oder 6 C-Atome und bedeutet demnach bevorzugt Ethyl, Propyl, Butyl, Pentyl, Hexyl.

Falls R¹ oder Y einen Alkenylrest bedeutet, so kann dieser geradkettig oder verzweigt sein. Vorzugsweise ist er verzweigt, hat 2, 3, 4, 5 oder 6 C-Atome und bedeutet demnach Vinyl, Allyl, Prop-1-enyl, But-1-(2- oder 3-)enyl, Pent-1-(2-, 3- oder 4-)enyl, Hex-1-(2-, 3-, 4- oder 5-)enyl.

Falls Y einen Alkoxyrest bedeutet, so ist dieser verzweigt oder geradkettig.

Vorzugsweise ist er geradkettig und bedeutet demnach bevorzugt Methoxy, Ethoxy, Propoxy, Butoxy, Pentyloxy oder Heptyloxy.

L¹ und L² sind vorzugsweise gleich und bedeuten F oder H, insbesondere F.

Unter diesen Verbindungen der Formel I sowie den Unterformeln sind diejenigen bevorzugt, in denen mindestens einer der darin enthaltenden Reste eine der angegebenen bevorzugten Bedeutungen hat.

In den Verbindungen der Formel I sind diejenigen Stereoisomeren bevorzugt, in denen die Ringe Cyc und Piperidin trans-1,4-disubstituiert sind. Diejenigen der vorstehend genannten Formeln. die eine oder mehrere Gruppen Pyd, Pyr, Dit und/oder Dio enthalten, umschließen jeweils die beiden 2,5-Stellungsisomeren.

Insbesondere bevorzugte Verbindungen der Formel I, welche eine Grippe der Formel 2 aufweisen, sind diejenigen der Teilformeln I2a bis I2x:

Cbu²-Phe-F I2a

Cbu²-PheF-F I2b

Cbu²-Phe-NCS I2c

Cbu²-PheF-NCS I2d

Cbu²-Phe-OCF₃ I2e

Cbu²-PheF-OCF₃ I2f

Cbu²-Phe-OCF₂H I2g

Cbu²-PheF-OCF₂H I2h

Cbu²-Cyc-Phe-F I2i

Cbu²-Cyc-PheF-F I2j

Cbu²-Cyc-PheF-NCS I2k

Cbu²-Cyc-Phe-NCS I2l

Cbu²-Cyc-Phe-OCF₃ I2m

Cbu²-Cyc-Phe-OCF₂H I2n

Cbu²-Cyc-PheF-OCF₂H I2o

Cbu²-Cyc-PheF-OCF₃ I2p

Cbu²-Phe-Alkyl I2q

Cbu²-Phe-OAlkyl I2r

Cbu²-Cyc-Phe-Alkyl I2s

Cbu²-Cyc-Phe-OAlkyl I2t

Cbu²-Phe-CN I2u

Cbu²-PheF-CN I2v

Cbu²-CycPhe-CN I2w

Cbu²-Cyc-PheF-CN I2x

Insbesondere bevorzugte Verbindungen der Formel I, welche eine Gruppe der Formel 5 aufweisen, sind diejenigen der Teilformeln I5a bis I5x:

Cbu⁵-Phe-F I5a

Cbu⁵-PheF-F I5b

Cbu⁵-Phe-NCS I5c

Cbu⁵-PheF-NCS I5d

Cbu⁵-Phe-OCF₃ I5e

Cbu⁵-PheF-OCF₃ I5f

Cbu⁵-Phe-OCF₂H I5g

Cbu⁵-PheF-OCF₂H I5h

Cbu⁵-Cyc-Phe-F I5i

Cbu⁵-Cyc-PheF-F I5j

Cbu⁵-Cyc-Phe-NCS I5k

Cbu⁵-Cyc-PheF-NCS I5l

Cbu⁵-Cyc-Phe-OCF₃- I5m

Cbu⁵-Cyc-PheF-OCF₃- I5n

Cbu⁵-Cyc-Phe-OCF₂H I5o

Cbu⁵-Cyc-PheF-OCF₂H I5p

Cbu⁵-Phe-CN I5q

Cbu⁵-PheF-CN I5r

Cbu⁵-CycPhe-CN I5s

Cbu⁵-Cyc-PheF-CN I5t

Cbu⁵-Cyc-Phe-OAlkyl I5u

Cbu⁵-Cyc-Phe-Alkyl I5v

Cbu⁵-Phe-OAlkyl I5w

Cbu⁵-Phe-Alkyl I5x

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Weiterhin können die Verbindungen der Formel I aus den entsprechenden 3-substituierten Cyclobutanonen durch Kondensation mit Methan-Derivaten nach C. Burton et al., Tetrahedron Lett. 29 (24), 3003-6 (1988) bzw. J. Fried, et al., Tetrahedron Lett. 25, 4329 (1984) in Gegenwart eines Phosphins hergestellt werden und anschließende Hydrierung (z.B. Schemen 1 bis 4).

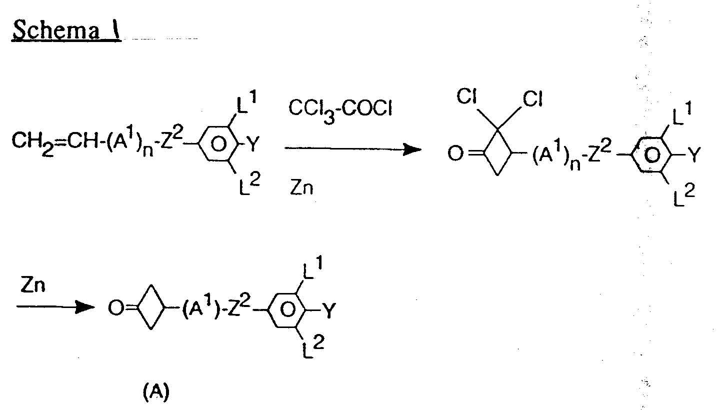

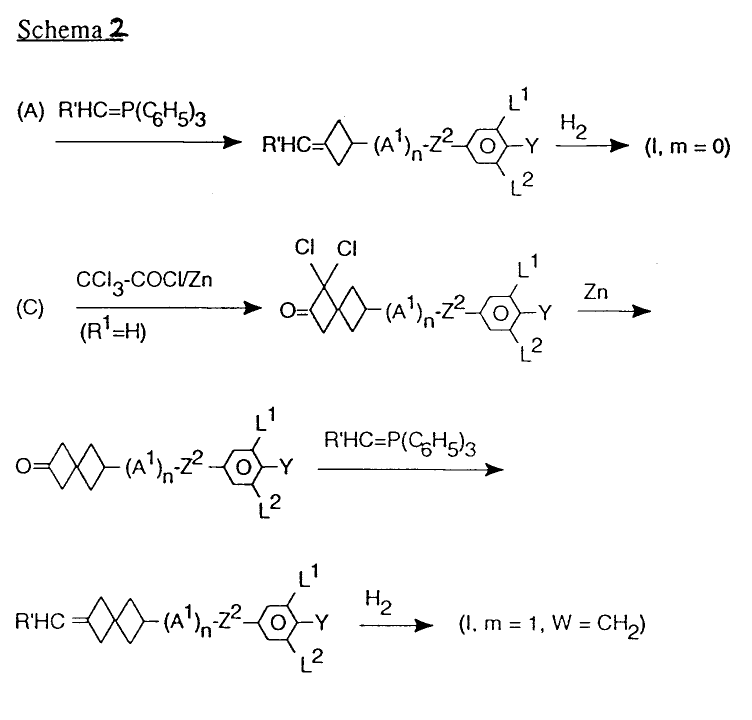

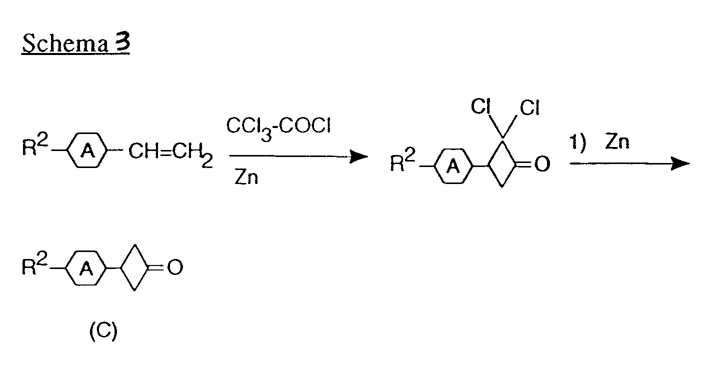

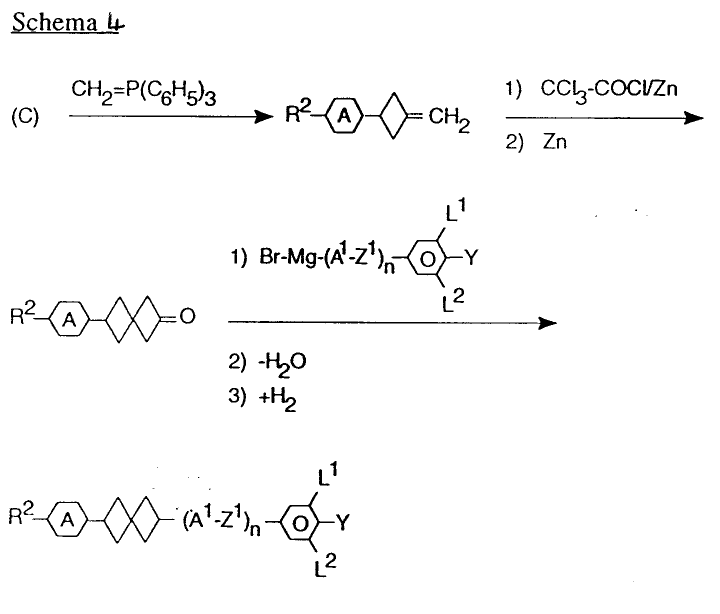

Weiterhin können die Verbindungen der Formel I hergestellt werden, indem man eine Verbindung, die sonst der Formel I entspricht, aber an Stelle von H-Atomen eine oder mehrere reduzierbare Gruppen und/oder C-C-Bindungen enthält, reduziert.

Als reduzierbare Gruppen kommen vorzugsweise Carbonylgruppen in Betracht, insbesondere Ketogruppen, ferner z.B. freie oder veresterte Hydroxygruppen oder aromatisch gebundene Halogenatome. Bevorzugte Ausgangsstoffe für die Reduktion entsprechen der Formel I, können aber an Stelle eines Cyclohexanringes einen Cyclohexenring oder Cyclohexanonring und/oder an Stelle einer -CH₂CH₂-Gruppe eine -CH=CH-Gruppe und/oder an Stelle einer -CH₂-Gruppe eine -CO-Gruppe und/oder an Stelle eines H-Atoms eine freie oder eine funktionell (z.B. in Form ihres p-Toluolsulfonats) abgewandelte OH-Gruppe enthalten.

Die Reduktion kann z.B. erfolgen durch katalytische Hydrierung bei Temperaturen zwischen etwa O° und etwa 200° sowie Drucken zwischen etwa 1 und 200 bar in einem inerten Lösungsmittel, z.B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z.B. PtO₂, PdO), auf einem Träger (z.B. Pd auf Kohle, Calciumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80 und 120°) oder Wolff-Kishner (mit Hydrazin, zweckmäßig in Gegenwart von Alkali wie KOH oder NaOH in einem hochsiedenden Lösungsmittel wie Diethylenglykol oder Triethylenglykol bei Temperaturen zwischen etwa 100 und 200°) zu den entsprechenden Verbindungen der Formel I, die Alkylgruppen und/oder -CH₂CH₂-Brücken enthalten, reduziert werden.

Weiterhin sind Reduktionen mit komplexen Hydriden möglich. Beispielsweise können Arylsulfonyloxygruppen mit LiAlH₄ reduktiv entfernt werden, insbesondere p-Toluolsulfonyloxymethylgruppen zu Methylgruppen reduziert werden, zweckmäßig in einem inerten Lösungsmittel wie Diethylether oder THF bei Temperaturen zwischen etwa 0 und 100°. Doppelbindungen können mit NaBH₄ oder Tributylzinnhydrid in Methanol hydriert werden.

Verbindungen der Formel I, die ansonsten der Formel I entsprechen, aber an Stelle von 1,4-Phenylenresten 1,4-Cyclohexenylenreste besitzen, können zum Beispiel mit DDQ (Dichlordicyanobenzochinon) in einem geeigneten Lösungsmittel oxidiert werden.

Ester der Formel I können auch durch Veresterung entsprechender Carbonsäuren (oder ihrer reaktionsfähigen Derivate), insbesondere der Formel IV, mit Alkoholen bzw. Phenolen (oder ihren reaktionsfähigen Derivaten), insbesondere der Formel V, oder nach der DCC-Methode (DCC = Dicyclohexylcarbodiimid) erhalten werden.

Die entsprechenden Carbonsäuren und Alkohole bzw. Phenole sind bekannt oder können in Analogie zu bekannten Verfahren hergestellt werden.

Als reaktionsfähige Derivate der genannten Carbonsäuren eignen sich insbesondere die Säurehalogenide, vor allem die Chloride und Bromide, ferner die Anhydride, z.B. auch gemischte Anhydride, Azide oder Ester, insbesondere Alkylester mit 1-4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls wie Natrium oder Kalium, in Betracht.

Die Veresterung wird vorteilhaft in Gegenwart eines inerten Lösungsmittels durchgeführt. Gut geeignet sind insbesondere Ether wie z.B. Diethylether, Di-n-butylether, THF, Dioxan oder Anisol, Ketone wie z. B. Aceton, Butanon oder Cyclohexanon, Amide wie z. B. DMF oder Phosphorsäurehexamethyltriamid, Kohlenwasserstoffe wie z.B. Benzol, Toluol oder Xylol, Halogenkohlenwasserstoffe wie z. B. Tetrachlorkohlenstoff, Dichlormethan oder Tetrachlorethylen und Sulfoxide wie z. B. Dimethylsulfoxid oder Sulfolan.

Zur Herstellung von Nitrilen der Formel I können entsprechende Säureamide, z.B. solche, in denen an Stelle des Restes CN eine CONH₂-Gruppe steht, dehydratisiert werden. Die Amide sind z.B. aus entsprechenden Estern oder Säurehalogeniden durch Umsetzung mit Ammoniak erhältlich. Als wasserabspaltende Mittel eignen sich beispielsweise anorganische Säurechloride wie SOCl₂, PCl₃, PCl₅, POCl₃, SO₂Cl₂, COCl₂, ferner P₂O₅, P₂S₅, AlCl₃ (z.B. als Doppelverbindung mit NaCl), aromatische Sulfonsäuren und Sulfonsäurehalogenide. Man kann dabei in Gegenwart oder Abwesenheit eines inerten Lösungsmittels bei Temperaturen zwischen etwa 0° und 150° arbeiten; als Lösungsmittel kommen z.B. Basen wie Pyridin oder Triethylamin, aromatische Kohlenwasserstoffe wie Benzol, Toluol oder Xylol oder Amide wie DMF in Betracht.

Zur Herstellung der vorstehend genannten Nitrile der Formel I kann man auch entsprechende Säurehalogenide, vorzugsweise die Chloride, mit Sulfamid umsetzen, zweckmäßig in einem inerten Lösungsmittel wie z. B. Tetramethylensulfon bei Temperaturen zwischen etwa 80° und 150°, vorzugsweise bei 120°. Nach üblicher Aufarbeitung kann man direkt die Nitrile isolieren.

Ether der Formel I sind durch Veretherung entsprechender Hydroxyverbindungen, insbesondere der Formel VI bzw. VII, vorzugsweise entsprechender Phenole, erhältlich, wobei die Hydroxyverbindung zweckmäßig zunächst in ein entsprechendes Metallderivat, z.B. durch Behandeln mit NaH, NaNH₂, NaOH, KOH, Na₂CO₃ oder K₂CO₃ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie z. B. Aceton, 1,2-Dimethoxyethan, DMF oder Dimethylsulfoxid oder auch mit einem Überschuß an wäßriger oder wäßrigalkoholischer NaOH oder KOH bei Temperaturen zwischen etwa 20° und 100°.

Zur Herstellung von Nitrilen der Formel I können auch entsprechende Chlor-, Brom- oder Jodverbindungen der Formel I mit einem Cyanid umgesetzt werden, vorzugsweise mit einem Metallcyanid wie z.B. NaCN, KCN oder Cu₂(CN)₂, z. B. in Gegenwart von Pyridin in einem inerten Lösungsmittel wie z. B. DMF oder N-Methylpyrrolidon bei Temperaturen zwischen 20° und 200°.

Verbindungen der Formel I, worin A¹ durch mindestens ein F-Atom und/oder eine CN-Gruppe substituiert ist, können auch aus den entsprechenden Diazoniumsalzen durch Austausch der Diazoniumgruppe gegen ein Fluoratom oder gegen eine CN-Gruppe, z.B. nach den Methoden von Schiemann oder Sandmeyer, erhalten werden.

Dioxanderivate bzw. Dithianderivate der Formel I werden zwecksmäßig durch Reaktion eines entsprechenden Aldehyds (oder eines seiner reaktionsfähigen Derivate) mit einem entsprechenden 1,3-Diol (oder einem seiner reaktionsfähigen Derivate) bzw. einem entsprechenden 1,3-Dithiol hergestellt vorzugsweise in Gegenwart eines inerten Lösungsmittels wie z.B. Benzol oder Toluol und/oder eines Katalysators, z.B. einer starken Säure wie Schwefelsäure, Benzol- oder p-Toluolsulfonsäure, bei Temperaturen zwischen etwa 20° und etwa 150°, vorzugsweise zwischen 80° und 120°. Als reaktionsfähige Derivate der Ausgangsstoffe eignen sich in erster Linie Acetale.

Die genannten Aldehyde und 1,3-Diole bzw. 1,3-Dithiole sowie ihre reaktionsfähigen Derivate sind zum Teil bekannt, zum Teil können sie ohne Schwierigkeiten nach Standardverfahren der Organischen Chemie aus literaturbekannten Verbindungen hergestellt werden. Beispielsweise sind die Aldehyde durch Oxydation entsprechender Alkohole oder durch Reduktion von Nitrilen oder entsprechenden Carbonsäuren oder ihrer Derivate, die Diole durch Reduktion entsprechender Diester und die Dithiole durch Umsetzung entsprechender Dihalogenide mit NaSH erhältlich.

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexan-carbonsäurephenyl- oder cyclohexyl-ester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexene, Cyclohexylcyclohexylcyclohexene, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)ethane, 1-Cyclohexyl-2-biphenylylethane, 1-Phenyl-2-cyclohexylphenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R'-L-E-R'' 1

R'-L-COO-E-R'' 2

R'-L-OOC-E-R'' 3

R'-L-CH₂CH₂-E-R'' 4

R'-L-C≡C-E-R'' 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, - Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, - Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R'' bedeuten in den Verbindungen der Teilformeln 1a, 2a, 3a, 4a und 5a jeweils unabhängig voneinander Alkyl, Alkenyl, Alkoxy, Alkenyloxy oder Alkanoyloxy mit bis zu 8 Kohlenstoffatomen. Bei den meisten dieser Verbindungen sind R' und R'' voneinander verschieden, wobei einer dieser Reste meist Alkyl oder Alkenyl ist. In den Verbindungen der Teilformeln 1b, 2b, 3b, 4b und 5b bedeutet R'' -CN, -CF₃, -OCF₃, -OCHF₂, F, Cl oder - NCS; R hat dabei die bei den Verbindungen der Teilformeln 1a bis 5a angegebene Bedeutung und ist vorzugsweise Alkyl oder Alkenyl. Aber auch andere Varianten der vorgesehenen Substituenten in den Verbindungen der Formeln 1, 2, 3, 4 und 5 sind gebräuchlich. Viele solcher Substanzen oder auch Gemische davon sind im Handel erhältlich. Alle diese Substanzen sind nach literaturbekannten Methoden oder in Analogie dazu erhältlich.

Die erfindungsgemäßen Medien enthalten vorzugsweise neben Komponenten aus der Gruppe der Verbindungen 1a, 2a, 3a, 4a und 5a (Gruppe 1) auch Komponenten aus der Gruppe der Verbindungen 1b, 2b, 3b, 4b und 5b (Gruppe 2), deren Anteile vorzugsweise wie folgt sind:
- Gruppe 1:: 20 bis 90 %, insbesondere 30 bis 90 %,
- Gruppe 2:: 10 bis 80 %, insbesondere 10 bis 50 %,
wobei die Summe der Anteile der erfindungsgemäßen Verbindungen und der Verbindungen aus den Gruppen 1 und 2 bis zu 100 % ergeben.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungegemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Auch ohne weitere Ausführungen wird davon ausgegangen, daß ein Fachmann die obige Beschreibung in weitesten Umfang nutzen kann. Die bevorzugten Ausführungsformen sind deswegen lediglich als beschreibende, keineswegs als in irgendeine Weise limitierende Offenbarung aufzufassen.

Die vollständige Offenbarung aller vor- und nachstehend aufgeführten Anmeldungen, Patente und Veröffentlichungen, sowie der korrespondierenden Anmeldung P 42 39 169, eingereicht am 21.11.92, sind durch Bezugnahme in diese Anmeldung eingeführt. mp. = Schmelzpunkt, cp. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Es bedeuten ferner:

K: Kristallin-fester Zustand, S: smektische Phase (der Index kennzeichnet den Phasentyp), N: nematischer Zustand, Ch: cholesterische Phase, I: isotrope Phase. Die zwischen zwei Symbolen stehende Zahl gibt die Umwandlungstemperatur in Grad Celsius an.
- DAST: Diethylaminoschwefeltrifluorid
- DCC: Dicyclohexylcarbodiimid
- DDQ: Dichlordicyanobenzochinon
- DIBALH: Diisobutylaluminiumhydrid
- HMTAP: Hexamethyltriaminophosphin
- KOT: Kalium-tertiär-butanolat
- PCC: Pyridiniumchlorochromat
- THF: Tetrahydrofuran
- TPP: Triphenylphosphin
- pTSOH: p-Toluolsulfonsäure

### Beispiel 1

### Darstellung von 3-(3,4,5-Trifluorphenyl)-1-methyl-cyclobutan

A) 3,4,5-Trifluor-vinylbenzol
   Ein Gemisch aus 1 mol 3,4,5-Trifluor-brombenzol und 3 l THF wird bei -70 °C mit 1 mol BuLi versetzt. Zu dem Reaktionsgemisch werden anschließend ein Gemisch aus 0,5 mol Zinkbromid in 1 l THF zugegeben und 30 Minuten bei -65 °C gerührt. Anschließend werden 1 mol Vinylbromid und 0,022 mol Nickel(II)chlorid/TPP hinzugegeben.
   Das Reaktionsgemisch wird 16 Stunden bei Raumtemperatur gerührt und wieüblich aufgearbeitet. Das erhaltene Styrol-Derivat wird ungereinigt weiterverarbeitet.
B) 3-(3,4,5-Trifluor-phenyl)-2,2-dichlorcyclobutanon
   Ein Gemisch aus 0,25 mol 1A, 23,0 g Zink-Kupfer (3 % Kupfer) und 800 ml Diethylether wird innerhalb 15 Minuten mit 0,25 ml Trichloracetylchlorid versetzt und anschließend 8 Stunden unter Rückfluß gerührt. Nach üblicher Aufarbeitung erhält man das Produkt, welches ungereinigt weiterverarbeitet wird.
C) 3-(3,4,5-Trifluo-phenyl)-cyclobutanon
   Ein Gemisch aus 0,144 mol 1B, 0,53 mol Zink-Pulver und 880 ml Eisessig wird 17 Stunden bei Raumtemperatur gerührt. Nach üblicher Aufarbeitung erhält man das Produkt, welches ungereinigt weiterverarbeitet wird.
D) 3-(3,4,5-(Trifluor-phenyl)-1-methylencyclobutan
   Ein Gemisch aus 0,15 mol Triphenylmethylenphosphin, 100 ml Tetraglyme und 0,13 mol 1C hinzugegeben und 16 Stunden bei Raumtemperatur gerührt. Nach üblicher Aufarbeitung und Destillation aus 100 ml Ethanol/Ethylacetat erhält man das reine Produkt.

### Beispiel 2

### 6-(3,4,5-Trifluorphenyl)-2-methylspiro[3.3]heptan

2A) 7-(3,4,5-Trifluorphenyl)-1,1-dichlorspiro[3.3]-heptan-2-on
   0,1 mol 1D wird gemäß Beispiel 1B mit 0,1 mol Trichloracetylchlorid umgesetzt. Nach üblicher Aufarbeitung erhält man das Produkt, welches ungereinigt weiterverarbeitet wird.
2B) 6-(3,4,5-Trifluorphenyl)-spiro[3.3]heptan-2-on
   Ein Gemisch aus 0,075 mol 2A und 0,25 mol Zink-Pulver wird gemäß Beispiel 1C umgesetzt.
2C) 6-(3,4,5-Trifluorphenyl)-2-methylenspiro[3.3]heptan
   0,05 mol 2B werden gemäß Beispiel 1D mit 0,06 mol Triphenylphosphin umgesetzt. Nach üblicher Aufarbeitung und Chromatographischer Reinigung erhält man das Produkt.
2D) 0.01 mol 2C werden unter Zusatz von 0.2 mmol Pd-C (10%ig) und 20 ml Toluol bei Raumtemperatur hydriert. Nach üblicher Aufarbeitung und Chromatographie erhält man das reine Produkt.

Analog werden hergestellt:
6-(3,4-Difluorphenyl)-2-methylspiro[3.3]heptan
6-(4-Trifluormethoxyphenyl)-2-methylspiro[3.3]heptan
6-(3,5-Difluor-4-difluormethoxyphenyl)-2-methylspiro[3.3]heptan
6-(3,4,5-Trifluorphenyl)-2-ethyl-spiro[3.3]heptan
6-(3,4,5-Trifluorphenyl)-2-propyl-spiro[3.3]heptan, K 9 I
6-(3,4,5-Trifluorphenyl)-2-butyl-spiro[3.3]heptan
6-(3,4,5-Trifluorphenyl)-2-pentyl-spiro[3.3]heptan, K 11 I
6-(3,4,5-Trifluorphenyl)-2-hexyl-spiro[3.3]heptan
6-(3,4,5-Trifluorphenyl)-2-heptyl-spiro[3.3]heptan
6-(3,4-Difluorphenyl)-2-ethyl-spiro[3.3]heptan
6-(3,4-Difluorphenyl)-2-propyl-spiro[3.3]heptan
6-(3,4-Difluorphenyl)-2-butyl-spiro[3.3]heptan
6-(3,4-Difluorphenyl)-2-pentyl-spiro[3.3]heptan
6-(3,4-Difluorphenyl)-2-hexyl-spiro[3.3]heptan
6-(3,4-Difluorphenyl)-2-heptyl-spiro[3.3]heptan
6-[trans-4-(3,4,5-Trifluorphenyl]-cyclohexyl]-2-methyl-spiro[3.3]heptan
6-[trans-4-(3,4,5-Trifluorphenyl]-cyclohexyl]-2-ethyl-spiro[3.3]heptan, K 62 I
6-[trans-4-(3,4,5-Trifluorphenyl]-cyclohexyl]-2-propyl-spiro[3.3]heptan, K 47 N (44.7) I
6-[trans-4-(3,4,5-Trifluorphenyl]-cyclohexyl]-2-butyl-spiro[3.3]heptan, K 56 I
6-[trans-4-(3,4,5-Trifluorphenyl]-cyclohexyl]-2-pentyl-spiro[3.3]heptan, K 55 N (41.5) I
6-[trans-4-(3,4,5-Trifluorphenyl]-cyclohexyl]-2-hexyl-spiro[3.3]heptan
6-[trans-4-(3,4,5-Trifluorphenyl]-cyclohexyl]-2-heptyl-spiro[3.3]heptan
6-(3,4,5-Trifluorbiphenyl-4'-yl)-2-methylspiro[3.3]heptan
6-(3,4,5-Trifluobiphenyl-4'-yl)-2-ethylspiro[3.3]heptan
6-(3,4,5-Trifluorbiphenyl-4'-yl)-2-propylspiro[3.3]heptan,
K 27 N (12.1) I
6-(4-Ethoxyphenyl)-2-propylspiro[3.3]heptan, K 6 N (4.9) I
6-(4-Ethoxyphenyl)-2-butylspiro[3.3]heptan
6-(4-Ethoxyphenyl)-2-ethylspiro[3.3]heptan
6-(4-Ethoxyphenyl)-2-methylspiro[3.3]heptan
6-(4-Methoxyphenyl)-2-propylspilo[3.3]heptan
6-(4-Methoxyphenyl)-2-butylspiro[3.3]heptan
6-(4-Methoxyphenyl)-2-ethylspiro[3.3]heptan
6-(4-Methoxyphenyl)-2-methylspiro[3.3]heptan
6-(4-Propoxyphenyl)-2-propylspiro[3.3]heptan
6-(4-Pentylphenyl)-2-propylspiro[3.3]heptan

### Beispiel 3

### 6-(3,4,5-Trifluorphenyl)-2-(trans-4-propyl-cyclohexyl)-spiro[3.3]heptan

4A) 3-(trans-4-Propylcyclohexyl)-2,2-dichlorcyclobutanon
   Ein Gemisch aus 0,25 mol trans-4-Propyl-1-vinylcyclohexan (hergestellt nach WO 88/02357) und 800 ml Diethylether wird gemäß Beispiel 1B mit Trichloracetylchlorid umgesetzt.
4B) 3-(trans-4-Propylcyclohexyl)-cyclobutanon
   0,1 mol 4A werden analog Beispiel 1C mit 0,4 mol Zink-Pulver behandelt.
4C) 3-(trans-4-Propylcyclohexyl)-1-methylencyclobutan
   0,05 mol 4B werden analog Beispiel 1D mit 0,07 mol Triphenylmethylenphosphin umgesetzt.
4D) 2-(tran-4-Propylcyclohexyl]-5,5-dichlorspiro[3.3]-heptan-6-on
   35 mmol 4C werden analog Beispiel 1B mit Trichloracetylchlorid umgesetzt.
4E) 2-(trans-4-Propylcyclohexyl)-spiro[3.3]-heptan-6-on
   25 mmol 4D werden analog Beispiel 1C mit Zink behandelt.
4F) 2-(trans-4-Propylcyclohexyl)-6-hydroxy-6-(3,4,5-trifluorphenyl)-spiro[3.3]heptan
   Ein Gemisch aus 15 mmol 3,4,5-Trifluorphenylmagnesiumbromid (hergestellt aus 3,4,5-Trifluorbrombenzol und Magnesiumspänen), 15 mmol 4E und 50 ml Diethylether werden 15 Stunden gerührt. Nach üblicher Aufarbeitung erhalt man das Produkt, das ohne Aufreinigung weiterverarbeitet wird.
4G) 2-(trans-4-Propylcyclohexyl)-6-(3,4,5-trifluorphenyl)-spiro[3.3]hept-5-en
   Ein Gemisch aus 12 mmol 4F, 100 ml Toluol und 0,5 g p-Toluolsulfonsaure wird 10 Stunden am Wasserabscheider erhitzt. Nach üblicher Aufarbeitung erhält man das Produkt, das ohne Aufreinigung weiterverarbeitet wird.
4H) 10 mmol 4G werden gemäß Beispiel 2D hydriert. Nach üblicher Aufarbeitung erhalt das Produkt K 46 N (61.4) I.

Analog werden hergestellt:
6-(3,4-Difluorphenyl)-2-(trans-4-propylcyclohexyl)-spiro[3.3]heptan
6-(3,4-Difluorphenyl)-2-(trans-4-ethlylcyclohexyl)-spirol[3.3]heptan
6-(3,4,5-Trifluorphenyl)-2-(tran-4-ethylcyclohexyl)-spiro[3.3]heptan
6-(4-Trifluormethoxyphenyl)-2-(trans-4-propylcyclohexyl)spiro [3.3]heptan, K 67 N (95.2) I
(6-(4-Difluormethoxy-3,5-difluorphenyl)-2-(trans-4-propylcyclohexyl)-spiro[3.3]heptan, K 43 N (77.5) I
6-(4-Cyancphenyl)-2-(trans-4-propylcyclohexyl)-spiro[3.3]heptan,
K 113 N (162) I
6-(4-Ethoxyphenyl)-2-trans-4-propylcyclohexyl)-spiro[3.3]heptan

### Beispiel 4

Zu einer Basismischung (B) bestehend aus 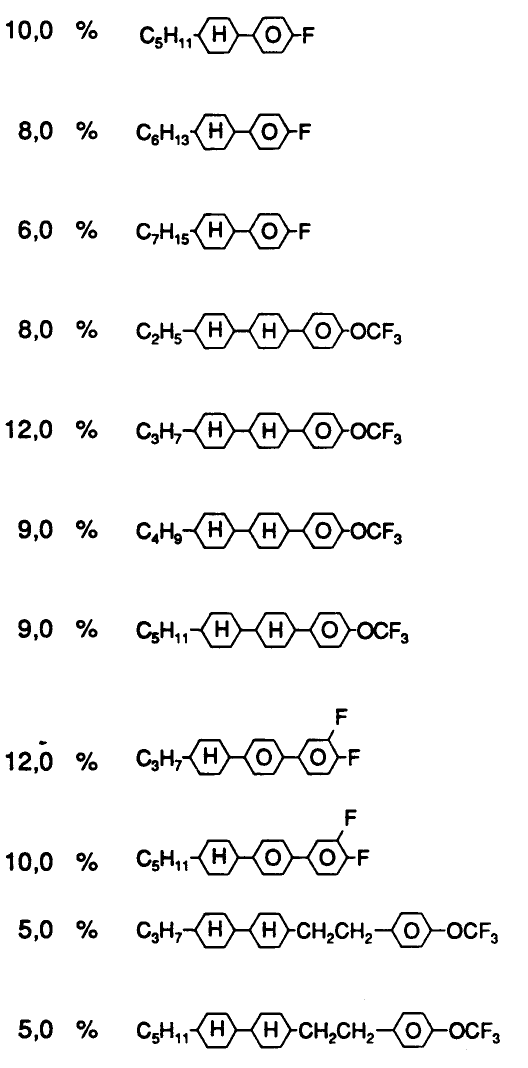 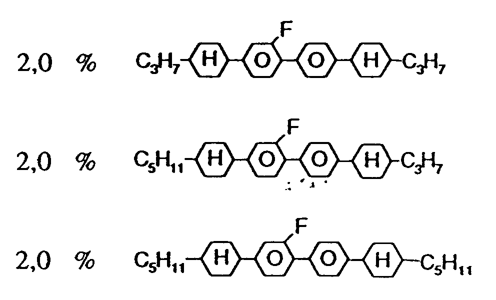 werden jeweils 10 der folgenden Verbindung gegeben: 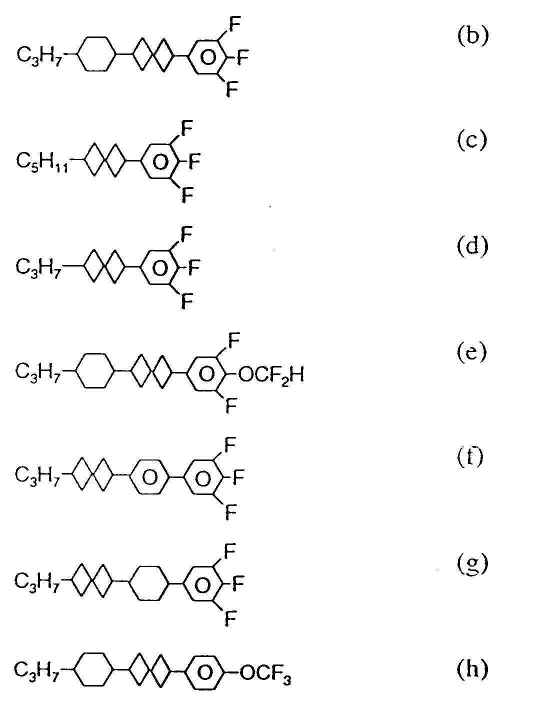 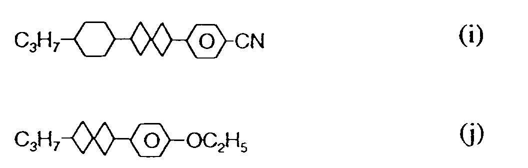 gegeben.

Die physikalischen Daten der so erhaltenen Mischungen können Tabelle I entnommen werden:

**Tabelle I**

| | **Klärpunkt (**°**C)** | Δε | Δ**n** | **Viskosität (mm**² · **s**⁻¹**)** |
|---|---|---|---|---|
| B | 91 | +5,2 | 0,094 | 15,0 |
| B + (b) | 86 | 5,5 | 0,092 | 14,8 |
| B + (c) | 69 | 5,0 | 0,084 | 14,2 |
| B + (d) | 68 | 5,2 | 0,084 | 13,9 |
| B + (e) | 87 | 5,4 | 0,092 | 15,6 |
| B + (f) | 83 | 5,8 | 0,097 | 14,9 |
| B + (g) | 83 | 5,5 | 0,091 | 15,0 |
| B + (h) | 90 | 5,2 | 0,094 | 14,4 |
| B + (i) | 98 | 6,2 | 0,102 | 17,7 |
| B + (j) | 80 | 4,5 | 0,092 | 14,2 |

Die erfindungsgemäßen Verbindungen erzielen in der Regel eine niedrigere optische Anisotropie, einen niedrigeren Klärpunkt und eine eine niedrigere Viskosität.

## Patentansprüche

1. Cyclobutan-Benzol-Derivate der Formel I, 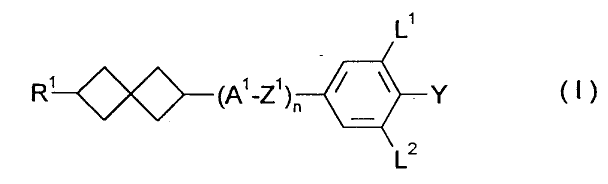 wobei
R¹ Alkyl oder Alkenyl mit 1 bis 16 C-Atomen, worin auch eine oder mehrere CH₂-Gruppen durch -O- ersetzt sein können, oder eine Gruppe der Formel 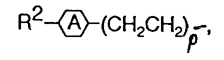 worin
R² Alkyl oder Alkenyl mit 1 bis 16 C-Atomen, 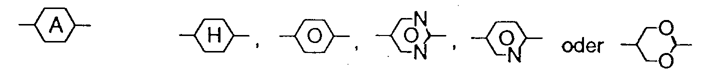 und
p 0 oder 1 bedeuten,
A¹ jeweils unabhängig voneinander unsubstituiertes oder durch 1 bis 2 Fluoratome substituiertes 1,4-Phenylen, worin auch eine oder zwei CH-Gruppen durch N ersetzt sein können, oder unsubstituiertes oder durch eine Cyanogruppe substituiertes 1,4-Cyclohexylen worin auch eine oder zwei CH₂-Gruppen durch O oder S ersetzt sein können, Thiadiazol-2,5-diyl oder 1,4-Bi-cyclo[2,2,2]-octylen bedeuten,
L¹ und L² jeweils unabhängig voneinander H oder F,
Z¹ -CO-O-, -O-CO-, -CH₂O-, -OCH₂-, -CH₂CH₂-, - C≡C- oder eine Einfachbindung,
Y NCS, Halogen oder eine durch mindestens ein Fluor- und/oder Chloratom substituierte Alkyl-, Alkoxy-, Alkenyl oder Alkenyloxygruppe mit 1 bis 8 C-Atomen, CN oder Alkyl oder Alkenyl mit bis zu 16 C-Atomen, worin auch 1 oder mehrere CH₂-Gruppen durch -O- umsetzt sein können,
n 0, 1 oder 2
bedeuten.

2. Derivate nach Anspruch 1, worin L¹ und L² F bedeuten.

3. Derivate nach Anspruch 1, worin Y F, OCF₃, OCF₂H oder CN bedeutet.

4. Derivate nach Anspruch 1 bis 3 der Formel II 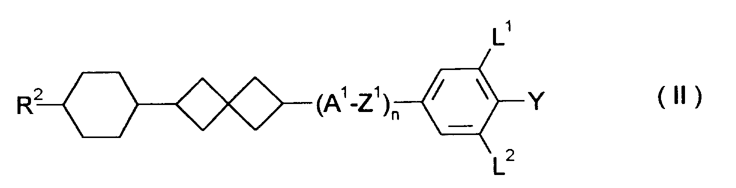 worin R², A¹, Z¹, n, Y, L¹ und L² die angegebene Bedeutung besitzen.

5. Derivate nach Anspruch 4 der Formel III 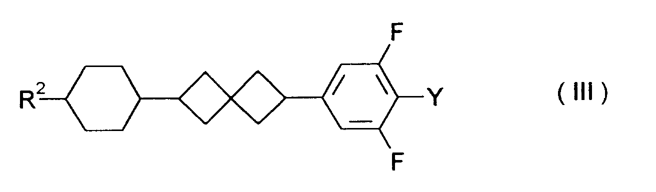 worin R² und Y die angegebene Bedeutung besitzen.

6. Flüssigkristallines Medium enthaltend mindestens zwei flüssigkristalline Komponenten, dadurch gekennzeichnet, daß es mindestens eine Verbindung der Formel I enthält.

7. Elektrooptische Anzeige enthaltend ein flüssigkristallines Medium nach Anspruch 6.

## Claims

1. Cyclobutane-benzene derivatives of the formula I 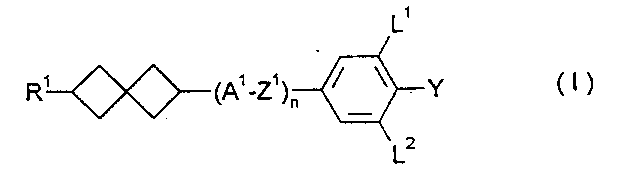 in which
R¹ is alkyl or alkenyl having 1 to 16 carbon atoms, in which, in addition, one or more CH₂ groups may be replaced by -O-, or is a group of the formula 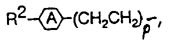 in which
R² is alkyl or alkenyl having 1 to 16 carbon atoms, 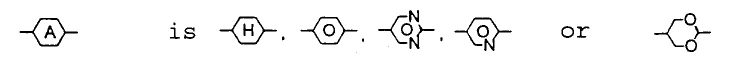 and
p is 0 or 1,
A¹ is at each occurrence, independently of the others, 1,4-phenylene which is unsubstituted or substituted by 1 to 2 fluorine atoms, in which, in addition, one or two CH groups may be replaced by N, or is 1,4-cyclohexylene which is unsubstituted or substituted by a cyano group, and in which, in addition, one or two CH₂ groups may be replaced by O or S, or is thiadiazole-2,5-diyl or 1,4-bicyclo[2.2.2]octylene,
L¹ and L² are each independently of one another H or F,
Z¹ is -CO-O, -O-CO-, -CH₂O-, -OCH₂-, -CH₂CH₂-, -C≡C- or a single bond,
Y is NCS, halogen or an alkyl, alkoxy, alkenyl or alkenyloxy group having 1 to 8 carbon atoms which is substituted by at least one fluorine and/or chlorine atom, is CN, or alkyl or alkenyl having up to 16 carbon atoms, in which, in addition, 1 or more CH₂ groups may be replaced by -O-,
n is 0, 1 or 2.

2. Derivatives according to Claim 1, in which L¹ and L² are F.

3. Derivatives according to Claim 1, in which Y is F, OCF₃, OCF₂H or CN.

4. Derivatives according to Claims 1 to 3, of the formula II 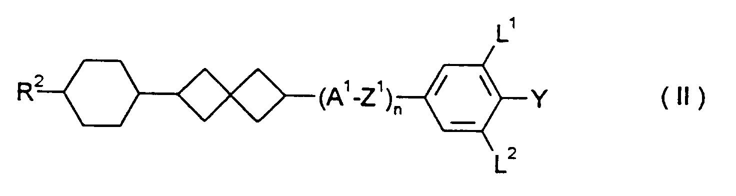 in which R², A¹, Z¹, n, Y L¹ and L² have the given meaning.

5. Derivatives according to Claim 4, of the formula III 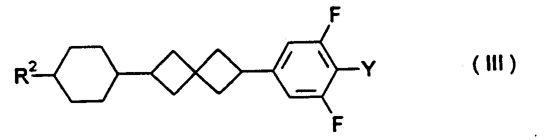 in which R² and Y have the given meaning.

6. Liquid-crystalline medium containing at least two liquid-crystalline components, characterized in that it contains at least one compound of the formula I.

7. Electrooptical display containing a liquid-crystalline medium according to Claim 6.

## Revendications

1. Dérivés de cyclobutane-benzène de formule I, 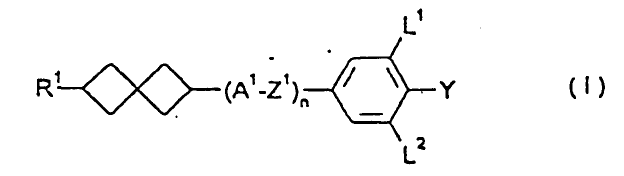 dans laquelle
R¹ représente un groupe alkyle ou alcényle ayant de 1 à 16 atomes de carbone, dans lequel un ou plusieurs groupes CH₂ peuvent également être remplacés par -O-,
ou un groupe de formule 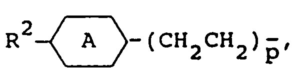 dans laquelle
R² représente un groupe alkyle ou alcényle ayant de 1 à 16 atomes de carbone, 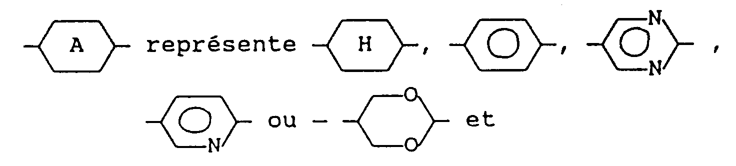
p représente 0 ou 1,
les radicaux A¹ représentent chacun, indépendamment les uns des autres, un radical 1,4-phénylène non substitué ou substitué par 1 ou 2 atomes de fluor, dans lequel un ou deux groupes CH peuvent également être remplacés par N, ou un radical 1,4-cyclohexylène non substitué ou substitué par un groupe cyano, dans lequel un ou deux groupes CH₂ peuvent également être remplacés par O ou S, ou bien le radical thiadiazole-2,5-diyle ou 1,4-bicyclo-[2,2,2]octylène,
L¹ et L² représentent chacun, indépendamment l'un de l'autre, H ou F,
Z¹ représente -CO-O-, -O-CO-, -CH₂O-, -OCH₂-, -CH₂CH₂-, -C≡C- ou une liaison simple,
Y représente NCS, un atome d'halogène ou un groupe alkyle, alcoxy, alcényle ou alcényloxy ayant de 1 à 8 atomes de carbone, substitué par au moins un atome de fluor et/ou un atome de chlore, CN ou un groupe alkyle ou alcényle ayant jusqu'à 16 atomes de carbone, dans lequel un ou plusieurs groupes CH₂ peuvent également être remplacés par -O-,
n représente 0, 1 ou 2.

2. Dérivés selon la revendication 1, dans lesquels L¹ et L² représentent F.

3. Dérivés selon la revendication 1, dans lesquels Y représente F, OCF₃, OCF₂H ou CN.

4. Dérivés selon l'une des revendications 1 à 3, de formule II 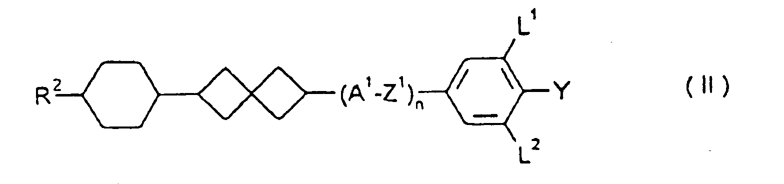 dans laquelle R², A¹, Z¹, n, Y, L¹ et L² ont les significations données.

5. Dérivés selon la revendication 4, de formule III 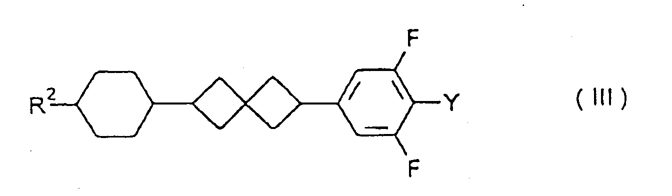 dans laquelle R² et Y ont les significations données.

6. Milieu à cristaux liquides, contenant au moins deux composants de type cristal liquide, caractérisé en ce qu'il contient au moins un composé de formule I.

7. Afficheur électro-optique contenant un milieu à cristaux liquides selon la revendication 6.
